# EUROPEAN PATENT APPLICATION

(11) **EP 4 578 384 A1**
(43) Date of publication of application: **02.07.2025**
(21) Application number: 23857048.5
(22) Date of filing: 20.07.2023
(51) Int. Cl.: A61B 5/151

(54) **BLOOD COLLECTION DEVICE AND BLOOD COLLECTION AND STORAGE DEVICE**

(30) Priority: 24.08.2022 JP 2022133104
(71) Applicant: Asahi Polyslider Company, Limited, Osaka 530-0005 (JP)
(72) Inventor: HANAFUSA, Hiroshi, Maniwa-shi, Okayama 719-3226 (JP)
(74) Representative: Murgitroyd & Company
(86) International application number: PCT/JP2023/026566
(87) International publication number: WO 2024/042932

(57) **Abstract**

Providing a blood collection device and a blood collection and storage device that can implement more appropriate blood collection with a simple construction. The blood collection device comprising a flow path guiding collected blood to a storage container comprising: a housing; a blade protruding from the housing and being movable to cut through a skin in one direction; a negative pressure generation cylinder generating negative pressure in the housing due to a plunger being movable; and a trigger member being accommodated in the housing and being actuated when blood collection starts, wherein the trigger member is engaged with the plunger and the blade to restrict both of the plunger and the blade from being movable, and is disengaged with both of the plunger and the blade to permit the blade and the plunger to be movable.

## Description

### Technical Field

The present disclosure relates to a blood collection device and a blood collection and storage device.

### Background Art

A blood collection device for collecting a bodily fluid such as blood from a subject is disclosed in Patent Literatures 1 to 3.

### Citation List

### Patent Literature

Patent Literature 1: Japanese Translation of PCT International Application Publication No. 2012-519052
Patent Literature 2: Japanese Translation of PCT International Application Publication No. 2018-534541
Patent Literature 3: Japanese Translation of PCT International Application Publication No. 2019-505282

### Summary of Invention

### Technical Problem

This type of blood collection device requires the collected blood to be injected into the device. Specifically, the blood is injected into an interior of the blood collection device by generating negative pressure inside the blood collection device in a state in which the blood collection device is brought into close contact with the skin of the subject.

A technique of using a "vacuum chamber having an internal pressure that is lower than atmospheric pressure" as described in Patent Literature 1 can be considered as one technique for generating negative pressure inside the blood collection device. However, the technique of using a vacuum chamber is troublesome in having to prepare in advance an inner pressure that is lower than atmospheric pressure.

A technique for creating a vacuum through a "change in volume when a plunger is movable" is described in Patent Literatures 2 and 3 as another technique for generating negative pressure inside the blood collection device. Specifically, Patent Literature 2 discloses creating a vacuum by a piercing element piercing the skin and using a plunger upon withdrawal of the piercing element. Patent Literature 3 discloses creating a vacuum within a sealed portion through a retraction operation after an actuation mechanism is deployed and a lancet extends to pierce the skin.

However, the blood collection devices disclosed in Patent Literatures 2 and 3 require a component serving as a trigger for the "operation of piercing the skin" and a component serving as a trigger for the "operation of generating a vacuum" to be prepared individually. As such, since the blood collection devices disclosed in Patent Literatures 2 and 3 require a large number of components, there is room for improvement from the perspective of manufacturing cost and/or the perspective of manufacturing efficiency.

An object of the present disclosure is to provide a blood collection device and a blood collection and storage device that can implement more appropriate blood collection with a simple construction.

### Solution to Problem

In order to solve the above-described problem, a blood collection device according to the present disclosure is a blood collection device including a flow path that guides collected blood to a storage container, the blood collection device comprising:
a housing;
a blade protruding from the housing and being movable to cut through a skin in one direction;
a negative pressure generation cylinder generating negative pressure in the housing due to a plunger being movable; and
a trigger member being accommodated in the housing and being actuated when blood collection starts, wherein
the trigger member is engaged with the plunger and the blade to restrict both of the plunger and the blade from being movable, and is disengaged with both of the plunger and the blade to permit the blade and the plunger to be movable.

A blood collection and storage device according to the present disclosure comprising:
the above blood collection device; and
a storage container being positioned at the direction of gravity side when the blood collection device is adhering to the skin, and stores blood collected by the blood collection device.

### Advantageous Effect of Invention

According to the present disclosure, it is possible to provide a blood collection device and a blood collection and storage device that can implement more appropriate blood collection with a simple construction.

### Brief Description of Drawings

[Figure 1] Figure 1A is a perspective view of a blood collection device according to the present disclosure as viewed from above, and Figure 1B is a perspective view of the blood collection device according to the present disclosure as viewed from below.
[Figure 2] Figure 2A is a perspective view of an interior of the blood collection device according to the present disclosure, and Figure 2B is a cross-sectional view cut along a line b-b in Figure 2A.
[Figure 3] Figure 3A is a cross-sectional view cut along a line i-i in Figure 1A, and Figure 3B is a cross-sectional view cut along the line i-i in Figure 1A in a mode in which a button is pressed down.
[Figure 4] Figures 4A to 4C are explanatory drawings for describing an operation of a sliding member in a blade according to a first embodiment.
[Figure 5] Figures 5A to 5E are explanatory drawings for describing an operation of the blade according to the first embodiment.
[Figure 6] Figures 6A to 6C are explanatory drawings for describing an operation of a sliding member in the blade according to a second embodiment.
[Figure 7] Figures 7A to 7E are explanatory drawings for describing an operation of the blade according to the second embodiment.
[Figure 8] Figure 8A is a cross-sectional view cut along a line ii-ii in Figure 1A, and
Figure 8B is a cross-sectional view cut along the line ii-ii in Figure 1A in a mode in which the button is pressed down.
[Figure 9] Figure 9 is an explanatory drawing for describing an operation of a negative pressure generation cylinder.
[Figure 10] Figure 10 is a perspective view of a trigger member.
[Figure 11] Figures 11A and 11B are explanatory drawings for describing a cut being made in the skin during blood collection.
[Figure 12] Figure 12A is an explanatory drawing for describing a state before attaching a storage container to the blood collection device according to the present disclosure, and
Figure 12B is an explanatory drawing for describing a state after attaching the storage container to the blood collection device according to the present disclosure.
[Figure 13] Figure 13 is an explanatory drawing for describing a state in which the blood collection device according to the present disclosure is attached to the skin.
[Figure 14] Figure 14 is an explanatory drawing for describing collecting blood using the blood collection device according to the present disclosure.
[Figure 15] Figure 15A is an explanatory drawing for describing a state in which blood has accumulated in the storage container, and Figure 15B is an explanatory drawing for describing a state in which the storage container has been removed from the blood collection device according to the present disclosure.

### Description of Embodiments

Hereinafter, a "blood collection device" and a "blood collection and storage device" according to the present disclosure will be described in detail. Although the embodiments are described with reference to the drawings as necessary, the illustrated contents are only schematically and exemplarily shown for understanding of the present disclosure, and the appearance, dimensional ratios, and the like may be different from an actual product.

The terms "vertical direction" and "horizontal direction" directly or indirectly as used in the present description respectively correspond to a vertical direction and a horizontal direction in the drawings. Unless otherwise specified, the same reference numerals or symbols indicate the same members/portions or the same semantic contents. It can be understood that a downward direction in a vertical direction (that is, a direction in which gravity acts) corresponds to a "downward direction" and the opposite direction thereof corresponds to an "upward direction". In the present description, terms expressing the relationship between elements (for example, "parallel", "perpendicular", and the like) and the shapes of elements do not express only a strict meaning, but rather are intended to include substantially equivalent ranges, for example, differences of several percent. Terms such as "provided" and "disposed" and terms derived therefrom are not limited to expressing a direct meaning and may express an aspect in which another element such as an interposing element is provided unless explicitly described otherwise.

First, the "blood collection device" according to an embodiment of the present disclosure will be described. The "blood collection device" as used in the present disclosure refers to a device for collecting blood from a subject in a broad sense, and refers to a device also including a flow path that guides the collected blood to a storage container in a narrow sense. The storage container that stores the collected blood may be built into the blood collection device or may be attachable to the blood collection device. In other words, the "blood collection and storage device" may be the blood collection device according to the present disclosure including the storage container.

A blood collection device 1 indicating an embodiment according to the present disclosure includes a housing 20, a blade 30, a negative pressure generation cylinder 40, and a trigger member 50. Hereinafter, each component will be described in detail with reference to the drawings.

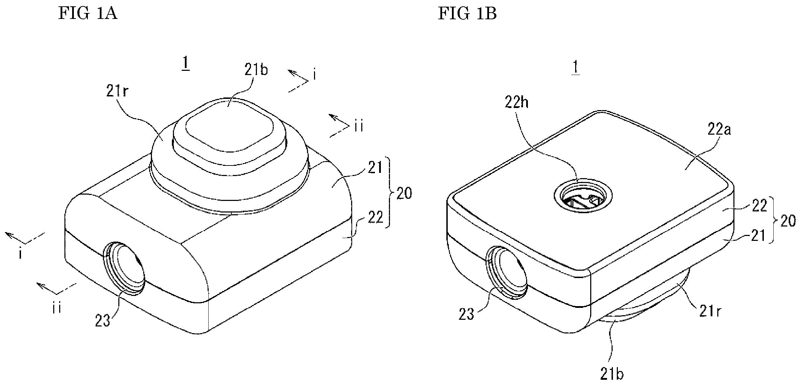

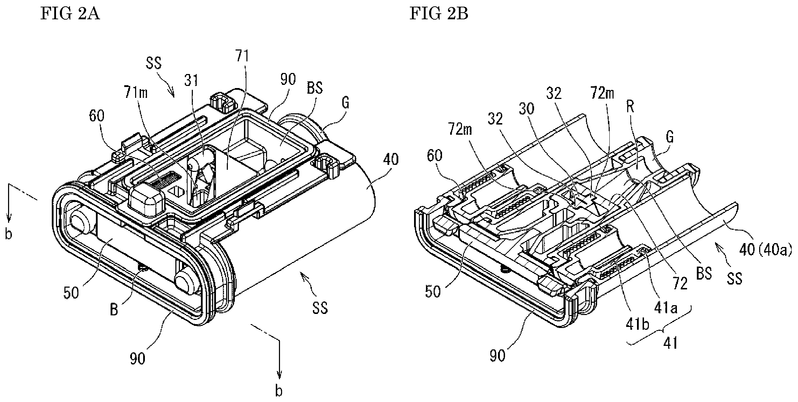

### - Description of Housing (See Figures 1 and 2 as Necessary) -

The housing 20 accommodates each component of the blood collection device 1. The housing of the illustrated example may have a square shape in its entirety and may be made of a resin material (for example, plastic). The housing 20 may be composed of, for example, a first body 21 and a second body 22 that can be coupled to each other. That is, a single housing 20 may be composed of the first body 21 and the second body 22.

The first body 21 may include a button 21b to be pressed down when the subject starts blood collection and a ridge portion 21r for providing a space that accommodates the pressed down button 21b. The button 21b may have a rectangular shape with rounded corners in plan view. The ridge portion 21r may also have a rectangular shape with rounded corners in plan view to correspond to the shape of the button 21b.

The second body 22 may include a facing surface that faces the skin of the subject. An adhesive member 22a may be provided on the facing surface. The adhesive member 22a may be any type of adhesive as long as the adhesive can adhere to the skin of the subject, but a hypoallergenic adhesive gentle to the skin is preferable. Appropriate blood collection can be performed by the blood collection device adhering to the skin of the subject through the adhesive member 22a.

An opening 22h may be provided in the facing surface for enabling protrusion of the blade 30 from the housing 20. Causing the blade 30 to protrude from the housing 20 through the opening 22h and making a cut in the skin of the subject makes it possible to collect blood.

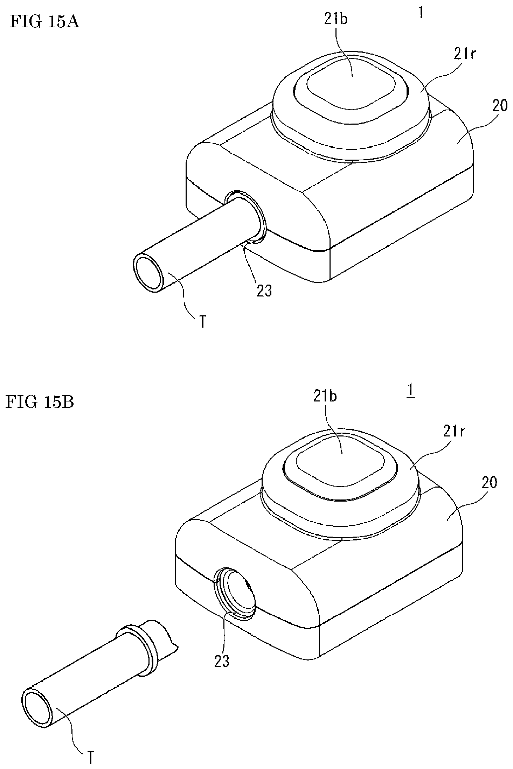

An attachment portion for attaching a storage container T (see Figure 15) that stores the collected blood may be provided in a lateral surface of the housing 20 obtained by coupling the first body 21 and the second body 22 to each other. As a preferred aspect of the attachment portion, an opening 23 may be provided that communicates with a flow path R (see Figure 2B) in through which the collected blood is guided. The storage container T may be freely attachable to the opening 23. More preferably, a gasket G for closely attaching the storage container T may be provided near the opening 23. The gasket G may be made of an elastically deformable resin material. By providing the gasket G near the opening 23, it is possible to prevent blood leakage when storing the collected blood in the storage container T.

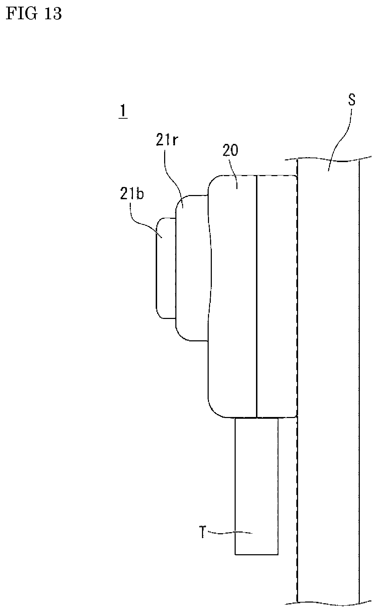

The attachment portion (the opening 23) is preferably disposed at the direction of gravity side (see also Figure 13 as necessary) of the housing 20 when the blood collection device 1 is adhering to the skin. In other words, the attachment portion is preferably disposed in a movable direction of a plunger 41 in the negative pressure generation cylinder 40 to be described below. By disposing the attachment portion in this way, the collected blood can be guided to the storage container T due to its own weight.

An interior of the housing 20 may include a blade space BS that accommodates at least the blade 30 and a cylinder space SS that accommodates the negative pressure generation cylinder 40 (see Figures 2A and B).

### · Blade Space That Accommodates Blade

The blade space BS may be disposed in a central portion of the housing 20. The blade space BS may communicate with the opening 22h in the facing surface described above. The blade space BS may communicate with the attachment portion (the opening 23) described above.

As a preferred aspect, negative pressure may be generated in the blade space BS by the negative pressure generation cylinder 40. That is, the blade space BS may act so as to draw in the collected blood due to negative pressure being generated. According to this aspect, it is possible to appropriately collect blood through the generation of negative pressure.

A circumference of the blade space BS may be hermetically sealed through an airtight retention member 90 in order to appropriately generate negative pressure in the blade space BS. As an example, the airtight retention member 90 may be made of an elastically deformable resin material. As an example, the airtight retention member 90 may be disposed above the blade space BS as shown in Figure 2A. The airtight retention member 90 may also be disposed at a lateral side of the blade space BS (as an example, at a lateral side of a side at which the trigger member 50 is disposed). In this way, airtightness of the blade space BS can be enhanced by disposing the airtight retention member 90 in the circumference of the blade space BS and hermetically sealing the blade space BS via the airtight retention member 90.

### · Cylinder Space That Accommodates Negative Pressure Generation Cylinder

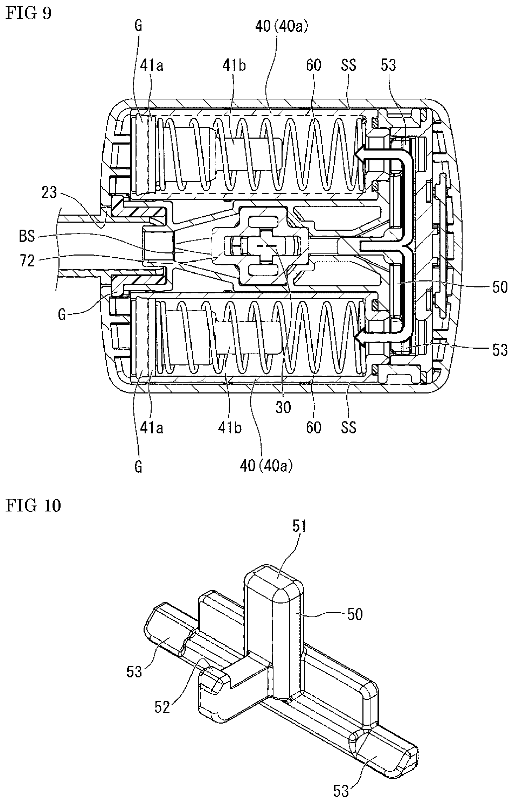

The cylinder space SS may be disposed at both sides of the blade space BS. That is, the cylinder space SS may be a pair of cylinder spaces SS. The cylinder space SS may communicate with the blade space BS at the side at which the trigger member 50 is disposed. As such, it is possible to generate negative pressure in the blade space BS through this communication, due to negative pressure being generated in the cylinder space SS through actuation of the negative pressure generation cylinder 40 (see Figure 9 as necessary).

The cylinder space SS may have a cylindrical shape corresponding to the cylindrical negative pressure generation cylinder 40 to be described below, in order to accommodate the cylindrical negative pressure generation cylinder 40. The cylinder space SS is preferably provided in an extending manner along a direction perpendicular to the direction of gravity when the blood collection device 1 is adhering to the skin. In other words, the cylinder space SS preferably extends in a direction in which the collected blood is guided to the storage container T. To specify the cylinder space SS from another perspective, the cylinder space SS preferably extends in a pushing direction of a compression coil spring 60 that allows the plunger 41 in the negative pressure generation cylinder 40 to be described below to be movable. By designing the cylinder space SS in this way, it is possible to effectively use an internal space of the housing 20. Note that in the present description, the negative pressure generation cylinder 40 is described as having a cylindrical shape, but the negative pressure generation cylinder 40 is not limited to this shape and may have, for example, a columnar shape.

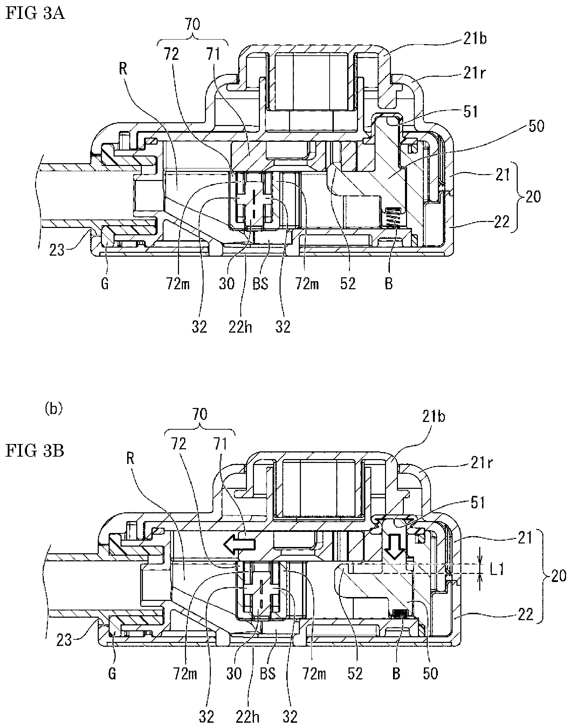

### - Description of Blade (See Figures 3 to 7 as Necessary) -

The blade 30 may include a metal blade for making a cut into the skin and a resin blade holding portion for holding the blade. Note that the resin blade holding member may be made of the same resin material as the housing 20 or may be made of a different material. The blade 30 is movable so as to protrude from the housing 20 during blood collection and make a cut in the skin in one direction. As a preferred aspect of the blade 30, the blade 30 may engage with a movable body 70 (a slider member 71 and a guide member 72) to be described below. As a preferred aspect of the blade 30, the blade 30 may include an end portion 31 having an engagement projection for engaging with the slider member 71 to be described below, and a shaft portion 32 that acts as a rotation shaft of the blade 30 and engages with the guide member 72. Note that although the shaft portion 32 acts as a rotation center of the blade 30, the shaft portion 32 may also shift along a guide groove 72m of the guide member 72 to be described below. Note that the shifting may be, specifically, a vertical motion.

The end portion 31 may extend from the blade 30 (more specifically, an end portion of the blade holding portion) along the pushing direction of the compression coil spring 60 used to make the slider member 71 movable (see Figure 2A). The end portion 31 may have a round columnar shape for allowing the blade 30 to perform a rotation operation. Note that the end portion 31 is not limited to having a round columnar shape as long as the blade 30 is allowed to perform the rotation operation smoothly.

The shaft portion 32 may extend from the blade 30 (more specifically, a lateral surface of the blade holding portion) along the pushing direction of the compression coil spring 60 used to make the slider member 71 movable. The shaft portion 32 may protrude in a round columnar shape from the lateral surface of the blade holding portion. That is, the shaft portion 32 may be a projection acting as an axis of a shaft rotation. Note that the shaft portion 32 is not limited to having a round columnar shape as long as a smooth shaft rotation is possible.

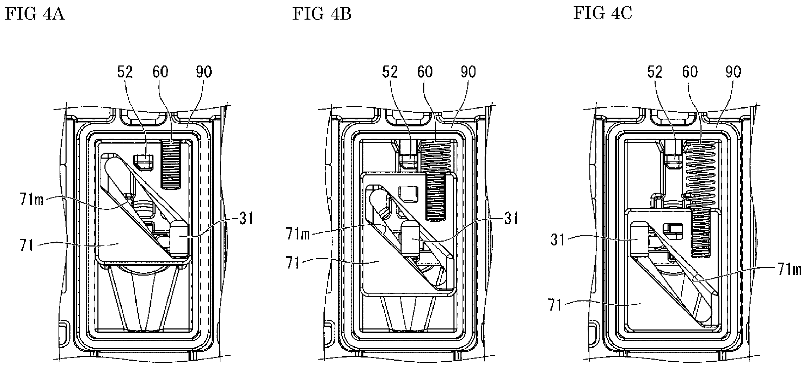

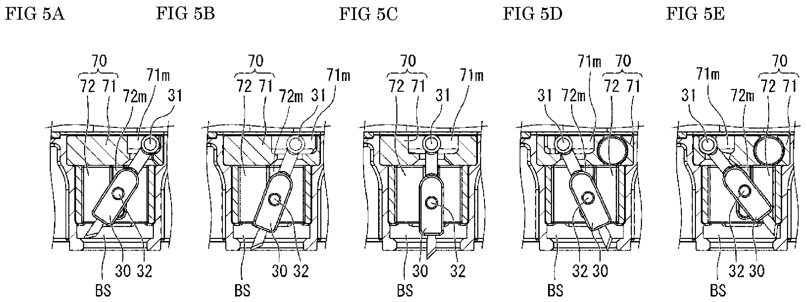

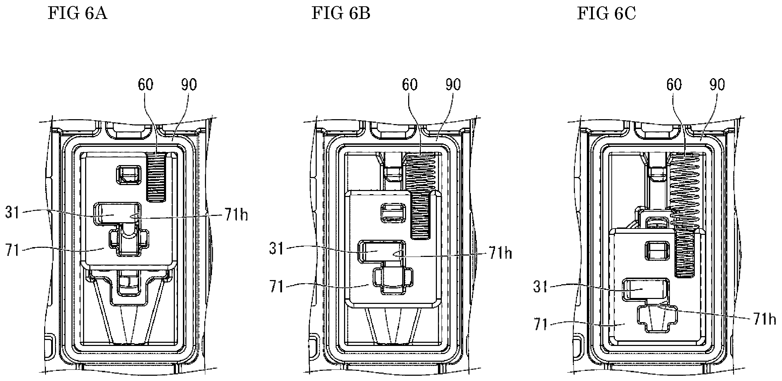

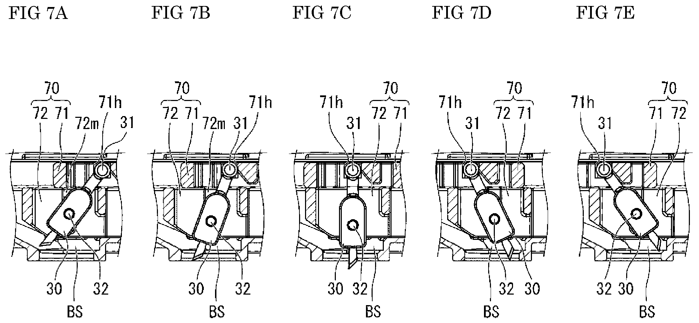

As a first embodiment of the blade 30, a direction in which the blade 30 makes a cut in the skin may be perpendicular to the direction of gravity (hereinafter, also referred to as a "horizontal cutting direction") when the blood collection device is adhering to the skin (see Figures 4 and 5). As a second embodiment of the blade 30, the direction in which the blade 30 makes a cut in the skin may be the direction of gravity (hereinafter, also referred to as a "vertical cutting direction") when the blood collection device is adhering to the skin (see Figures 6 and 7). The blade according to the first embodiment and the blade according to the second embodiment will be described in this order.

### · Configuration of Blade According to First Embodiment

The compression coil spring 60 that is compressed in advance may be used to make the blade 30 movable (see Figures 4A to C). That is, the compression coil spring 60 may be compressed toward the side at which the trigger member 50 is disposed before blood collection. In other words, the compression coil spring 60 may be compressed along the extension direction of the cylinder space SS. The blade 30 is movable by releasing the compression of the compression coil spring 60.

As a preferred aspect of the compression coil spring 60, the pushing direction of the compression coil spring 60 may be perpendicular to a direction in which the housing approaches the skin. In other words, the pushing direction may be parallel to a surface of the skin. Through such a pushing direction of the compression coil spring 60, it is possible to prevent a pushing force from being applied in the direction perpendicular to the skin and to reduce a burden on the skin.

The blade 30 being movable may be achieved by the movable body 70 rotating the blade 30 through the pushing force (biasing force) generated from the compression coil spring 60. In other words, a cut may be made in the skin in a predetermined direction (in the first embodiment, the horizontal cutting direction) due to the blade 30 rotating. Note that the movable body 70 may be made of a resin material (as an example, plastic) similarly to the housing 20 or may be made of another material.

The movable body 70 may include the slider member 71 that holds the end portion 31 and is movable through the pushing force of the compression coil spring 60, and the guide member 72 including the guide groove 72m that accommodates the shaft portion 32 for allowing the blade to perform the shaft rotation (see Figures 3 to 5). The blade 30 may make a cut in the skin in the predetermined direction through each of the slider member 71 and the guide member 72 being movable. Hereinafter, the slider member 71 and the guide member 72 will be described in detail.

### · Slider Member (See Figure 4)

The slider member 71 is a plate-shaped member having a substantially rectangular shape in plan view, and may include an accommodation portion that accommodates the compression coil spring 60 in a corner portion thereof, a groove 71m formed in a diagonal line for allowing the end portion 31 to perform a slide movement, and an engagement portion that the trigger member 50 (a blade lock piece 52) engages with. In other words, "in a diagonal line" as used in the present description may be a direction forming an angle with respect to the extension direction of the negative pressure generation cylinder 40. In other words, "in a diagonal line" as used in the present description may be a direction forming an angle with respect to the compression direction of the compression coil spring 60 allowing the slider member 71 to be movable. Note that the direction forming an angle may be an angle at which the end portion 31 can appropriately perform the slide movement as a specific aspect of the "direction forming an angle", and may be an acute angle as an example.

The groove 71m may be a through-groove. As a preferred aspect of the groove 71m, an overhang may be provided in the groove 71m for allowing the end portion 31 to perform the slide movement, the overhang being in contact with the end portion 31. That is, the end portion 31 may be capable of performing the slide movement while being held by the overhang of the through-groove.

### · Guide Member (See Figure 5)

The guide member 72 may be disposed below the slider member 71. The guide member 72 itself may be fixed inside the blade space BS. That is, the guide member 72 need not be movable inside the blade space BS. The shaft portion 32 of the blade may be accommodated in the guide groove 72m.

As a specific shape of the guide groove 72m, the guide groove 72m may be provided parallel to the direction in which the housing 20 approaches the skin. In other words, the guide groove 72m may be provided along a height direction of the housing 20. By forming the guide groove 72m in this way, the blade 30 can be allowed to be movable toward the skin.

### · Operation Description of Blade According to First Embodiment

An operation of the blade 30 and the movable body 70 (the slider member 71 and the guide member 72) according to the first embodiment will be described with reference to Figures 4 and 5.

Before blood collection, the slider member 71 is locked by the trigger member 50 (the blade lock piece 52). The compression coil spring 60 is in a compressed state toward the trigger member 50 side. Upon releasing the locking of the trigger member 50 (the blade lock piece 52) in this state, the slider member 71 performs the slide movement in a direction moving away from the trigger member 50 (from an upper side to a lower side of the drawings) through the pushing force (biasing force) generated from the compression coil spring 60. At this time, the end portion 31 moves from a right side toward a left side of the drawings in the illustrated example along the groove 71m in the diagonal line (Figures 4A to C).

On the other hand, the shaft portion 32 of the blade accommodated in the guide groove 72m is movable in conjunction with the slide movement of the slider member 71. Specifically, the shaft portion 32 performs a vertical motion in the vertical direction. According to the illustrated example (Figure 5A), the blade 30 is accommodated in the housing 20 when the slider member 71 is locked by the trigger member 50 (the blade lock piece 52). Upon releasing the locking of the trigger member 50 (the blade lock piece 52), the shaft portion 32 of the blade moves downward along the guide groove 72m in conjunction with the slide movement of the slider member 71 (Figure 5B). When the end portion 31 is positioned at a center of the groove 71m of the slider member 71 (that is, when the end portion 31 is positioned at a center of the blade space BS in a direction perpendicular to the slide direction), the shaft portion 32 of the blade 30 then moves to a position that is the lowest under the guide groove 72m and the blade 30 protrudes the most from the housing 20 as illustrated in Figure 5C. As the slide movement of the slider member 71 progresses, the shaft portion 32 of the blade moves upward along the guide groove 72m and the blade 30 is accommodated in the housing 20 (Figure 5D). When the compression coil spring 60 is fully stretched, the slide movement of the slider member 71 stops and the cutting by the blade ends (Figure 5E). Note that the blood collection device according to the present disclosure may be a disposable device. That is, the blood collection device may be a single-use device.

Through the above operation, the blade 30 is movable so as to perform the shaft rotation around the shaft portion 32, and a cut is made in the skin in the predetermined direction (in the first embodiment, the horizontal cutting direction) due to the blade 30 performing the shaft rotation.

### · Configuration of Blade According to Second Embodiment

As an aspect of a blade making a cut in the skin in a different direction than the blade according to the first embodiment, the blade shown in Figures 6 and 7 is provided. That is, a cut may be made in the skin in the vertical cutting direction due to the blade 30 performing the shaft rotation. Hereinafter, the movable body 70 according to the second embodiment will be described, but description overlapping with the first embodiment will be omitted as necessary.

### · Slider Member (See Figure 6)

The slider member 71 is a plate-shaped member having a substantially rectangular shape in plan view, and may include the accommodation portion that accommodates the compression coil spring 60 in the corner portion thereof, a holding portion 71h that holds the end portion 31, and the engagement portion that the trigger member 50 engages with.

The holding portion 71h may have a shape that matches the shape of the end portion 31 so as to fit into the end portion 31 having a round columnar shape, and, specifically, may be a through-hole. As a preferred aspect of the holding portion 71h, an overhang may be provided in the holding portion 71h for holding the end portion 31. That is, the end portion 31b may be held in the holding portion 71h by the overhang.

### · Guide Member (See Figure 7)

The guide member 72 may be disposed below the slider member 71. The guide member 72 itself may be fixed inside the blade space BS. That is, the guide member 72 need not be movable inside the blade space BS. The shaft portion 32 of the blade may be accommodated in the guide groove 72m.

As a specific shape of the guide groove 72m, the guide groove 72m may be provided parallel to the direction in which the housing 20 approaches the skin. In other words, the guide groove 72m may be provided along the height direction of the housing. By forming the guide groove 72m in this way, the blade 30 can be allowed to be movable toward the skin.

### · Operation Description of Blade According to Second Embodiment

An operation of the blade 30 and the movable body 70 (the slider member 71 and the guide member 72) according to the second embodiment will be described with reference to Figures 6 and 7.

Before blood collection, the slider member 71 is locked by the trigger member 50 (the blade lock piece 52). The compression coil spring 60 is in a compressed state toward the trigger member 50 side. Upon releasing the locking of the trigger member 50 (the blade lock piece 52) by the slider member 71 in this state, the slider member 71 performs the slide movement in the direction moving away from the trigger member 50 (from an upper side to a lower side of the drawings) through the pushing force (biasing force) generated from the compression coil spring 60. At this time, the end portion 31 is movable from the upper side toward the lower side of the drawings in the illustrated example in conjunction with the sliding of the slider member 71 (see Figures 6A to C). More precisely, the end portion 31 is movable in the same direction as the pushing direction of the compression coil spring 60 in conjunction with the slide movement of the slider member 71.

On the other hand, the shaft portion 32 of the blade accommodated in the guide groove 72m is movable in conjunction with the slide movement of the slider member 71. Specifically, the shaft portion 32 performs a vertical motion in the vertical direction. According to the illustrated example (Figure 7A), the blade 30 is accommodated in the housing 20 when the slider member 71 is locked by the trigger member 50 (the blade lock piece 52). Upon releasing the locking of the trigger member 50 (the blade lock piece 52), the shaft portion 32 of the blade 30 moves downward in the drawings along the guide groove 72m in conjunction with the slide movement of the slider member 71 (Figure 7B). When the end portion 31 is positioned at the center of the blade space BS in the slide direction, the shaft portion 32 of the blade 30 then moves to the position that is the lowest under the guide groove 72m and the blade 30 protrudes the most from the housing 20 as illustrated in Figure 7C. As the slide movement of the slider member 71 progresses, the shaft portion 32 of the blade 30 moves upward and the blade 30 is accommodated in the housing 20 (Figure 7D). When the compression coil spring 60 is fully stretched, the slide movement of the slider member 71 stops and the cutting by the blade ends (Figure 7E). Note that the blood collection device according to the present disclosure may be a disposable device. That is, the blood collection device may be a single-use device.

Through the above operation, the blade 30 is movable so as to perform the shaft rotation around the shaft portion 32, and a cut is made in the skin in the predetermined direction (in the second embodiment, the vertical cutting direction) due to the blade 30 performing the shaft rotation.

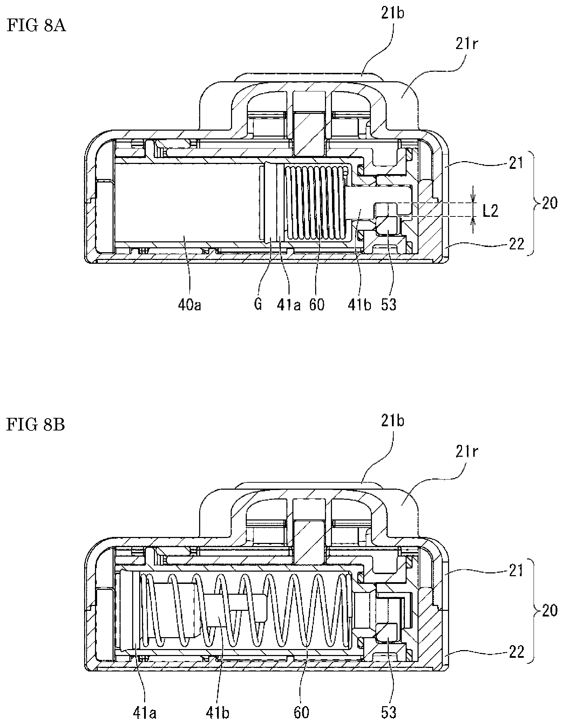

### - Description of Negative Pressure Generation Cylinder (See Figures 8 and 9) -

The negative pressure generation cylinder 40 generates negative pressure in the housing 20 due to the plunger 41 being movable during blood collection. As a preferred aspect of the negative pressure generation cylinder 40, the negative pressure generation cylinder 40 may include a cylinder body 40a having a cylindrical shape that extends along the movable direction of the plunger 41, and the plunger 41 that causes a change in volume by moving inside the cylinder body 40a.

The plunger 41 may include a disk portion 41a having a diameter substantially equal to an inner diameter of the cylinder body 40a, and a shaft core 41b that protrudes from the disk portion 41a toward the trigger member 50. The gasket G for closely attaching the disk portion 41a to an inner wall of the cylinder body 40a may be provided at the disk portion 41a. The shaft core 41b may include an engagement groove for engaging with the trigger member 50 (a plunger lock piece 53). The compression coil spring 60 may be attached to the shaft core 41b.

The compression coil spring 60 that is compressed in advance may be used to make the negative pressure generation cylinder 40 movable. That is, the compression coil spring 60 may be compressed toward the side at which the trigger member 50 is disposed before blood collection. It is possible to make the plunger 41 movable, cause a change in volume inside the cylinder body 40a, and generate negative pressure by releasing the compression of the compression coil spring 60.

As a preferred aspect of the compression coil spring 60, the pushing direction of the compression coil spring 60 may be perpendicular to the direction in which the housing approaches the skin. In other words, the pushing direction may be parallel to the surface of the skin. Through such a pushing direction of the compression coil spring 60, it is possible to prevent the pushing force from being applied in the direction perpendicular to the skin and to reduce the burden on the skin.

### · Operation Description of Negative Pressure Generation Cylinder

Before blood collection, the plunger 41 is locked by the trigger member 50 (the plunger lock piece 53) through the engagement groove. The compression coil spring 60 is in the compressed state toward the trigger member 50 side. Upon releasing the locking of the trigger member 50 (the plunger lock piece 53) in this state, the plunger 41 is movable in the direction moving away from the trigger member 50 through the pushing force (biasing force) generated from the compression coil spring 60. More precisely, the volume at the plunger 41 side in the cylinder body 40a gradually increases due to the plunger 41 being movable and negative pressure is generated.

It is possible to generate negative pressure in the blade space BS that communicates with the cylinder space SS, due to negative pressure being generated by the negative pressure generation cylinder 40. Note that upon the compression coil spring 60 being fully stretched, the volume increase at the plunger 41 side in the cylinder body 40a ends and the negative pressure generation ends.

### - Description of Trigger Member (See Figure 10) -

The trigger member 50 is locked to the plunger 41 and the blade 30 to restrict both the plunger 41 and the blade 30 from being movable before blood collection, and permits the blade 30 and the plunger 41 to be movable during blood collection by releasing the locking of both the plunger and the blade. Note that the trigger member 50 may be made of the same resin material as the housing 20 or may be made of a different material.

As a preferred aspect of the trigger member 50, the trigger member 50 may include an abutment portion 51 that abuts the button 21b of the housing, the blade lock piece 52 that locks the movability of the blade, and the plunger lock piece 53 that locks the movability of the plunger.

The abutment portion 51 may be provided so as to extend in the direction in which the housing 20 approaches the skin. The abutment portion 51 may be pressed downward in conjunction with the button 21b of the housing being pressed down. Note that a spring B (Figure 2A) having a repelling force that resists the button 21b of the housing from being pressed down may be provided below the abutment portion 51. Taking into consideration that the blood collection device according to the present disclosure may be a disposable device, the button 21b need not be restored from being pressed down even when the repelling by the spring B occurs. That is, the button 21b may be accommodated in the ridge portion 21r due to the button 21b being pressed down.

The blade lock piece 52 may be provided so as to protrude from a lateral surface of the abutment portion 51 toward the blade 30. The blade lock piece 52 may be lockable to the engagement portion of the slider member 71 described above. The locking of the blade lock piece 52 to the engagement portion of the slider member 71 may be released due to being pressed downward in conjunction with the button 21b of the housing being pressed down.

The plunger lock piece 53 may be provided so as to extend toward each negative pressure generation cylinder 40. More precisely, each plunger lock piece 53 is disposed on the same line. To specify each plunger lock piece 53 from another perspective, the line connecting the plunger lock pieces 53 in plan view may be perpendicular to a direction in which the blade lock piece 52 protrudes. To specify each plunger lock piece 53 from yet another perspective, the line connecting the plunger lock pieces 53 may be perpendicular to the direction in which the housing 20 approaches the skin.

As a further preferred aspect of the trigger member 50, a length L1 (Figure 3B) of the blade lock piece 52 may be shorter than a length L2 (Figure 8B) of the plunger lock piece 53. With such a configuration, the locking of the blade 30 by the blade lock piece 52 is released first and the locking of the plunger 41 by the plunger lock piece 53 is released after a cut is made in the skin with the blade 30. That is, it is possible to generate negative pressure in the blade space BS through the negative pressure generation cylinder 40 and to inject the collected blood into the housing 20 after making a cut into the skin with the blade 30.

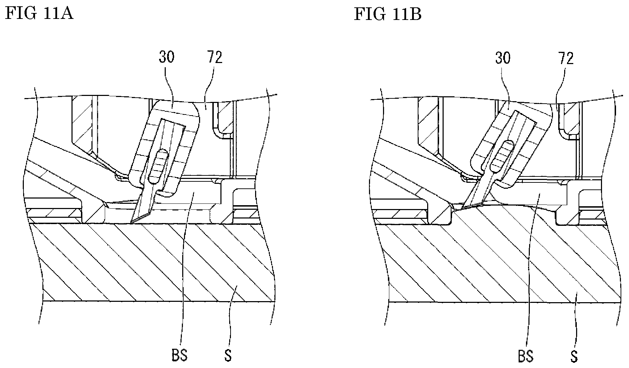

A reason why releasing the locking of the blade 30 first and subsequently releasing the locking of the negative pressure generation cylinder 40 as described above is useful will be described with reference to Figure 11.

When the locking of the blade 30 is released first and the locking of the negative pressure generation cylinder 40 is released subsequently, a skin S entering the blade space BS is prevented since negative pressure has not been generated in the blade space BS when making a cut in the skin S with the blade 30, as shown in Figure 11A. When the skin S does not enter the blade space BS, it is possible to maintain a depth at which the blade 30 enters the skin S at a fixed depth.

On the other hand, when the locking of the negative pressure generation cylinder 40 is hypothetically released first and the locking of the blade 30 is released subsequently, the blade 30 makes a cut into the skin S in a state in which negative pressure has been generated in the blade space BS through the negative pressure generation cylinder 40, as shown in Figure 11B. That is, a cut is made into the skin S in a state in which the skin S has entered the blade space BS. Since a firmness of the skin S is different for each subject, an amount of the skin S entering the blade space BS is different for each subject. In this case, the depth at which the blade 30 enters the skin S is different for each subject and a cut is not made consistently in the skin S with the blade 30.

According to the above description, as a preferred aspect of the trigger member 50, the locking of the blade 30 is preferably released first and the locking of the negative pressure generation cylinder 40 is released subsequently, due to the length L1 (Figure 3B) of the blade lock piece 52 being shorter than the length L2 (Figure 8B) of the plunger lock piece 53.

### - Description of Method for Using Blood Collection Device -

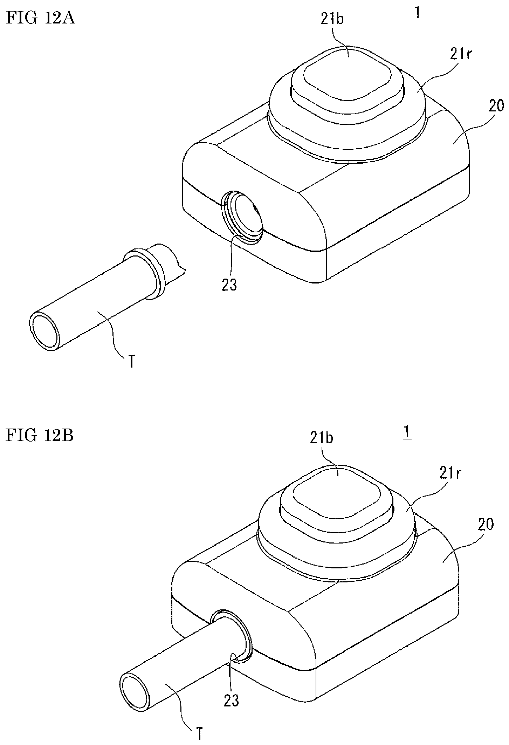

A method for using the blood collection device according to the present disclosure will be described with reference to Figures 12 to 15. First, the storage container T that stores the collected blood is prepared (Figure 12A). An external shape of the storage container T may be made to fit together with the attachment portion (the opening 23) of the housing described above.

After the storage container T is prepared, the storage container T is attached to the attachment portion (the opening 23) of the blood collection device 1 (Figure 12B). It is possible to closely attach the blood collection device 1 and the storage container T by providing the gasket G at the attachment portion (the opening 23).

After attaching the storage container T to the blood collection device 1, the blood collection device 1 is caused to adhere to the skin (Figure 13). By providing the adhesive member 22a on the facing surface of the blood collection device 1 that faces the skin of the subject, it is possible to appropriately cause the blood collection device to adhere to the skin of the subject. By causing the blood collection device to adhere to the skin, it is possible to hermetically seal the blade space BS since the opening 22h that communicates with the blade space BS is covered by the skin.

When causing the blood collection device 1 to adhere to the skin S, the blood collection device 1 preferably adheres to the skin S so that the storage container T attached to the blood collection device 1 is as the side at which gravity acts (lower side of the vertical direction). By causing the blood collection device 1 to adhere to the skin S in this way, the blood collected with the blood collection device 1 can be guided to the storage container T due to its own weight.

After causing the blood collection device 1 to adhere to the skin S, the button 21b provided to the housing 20 is pressed down. Before pressing down the button 21b, the trigger member 50 is locked to the plunger 41 and the blade 30 to restrict both the plunger 41 and the blade 30 from being movable. In this state, the trigger member 50 moves in the direction approaching the skin S by pressing down the button 21b. In conjunction therewith, the locking of both the plunger 41 and the blade 30 is released, and the blade 30 and the plunger 41 are permitted to be movable.

More specifically, due to the locking of the blade 30 being released by the blade lock piece 52 of the trigger member 50, the compression coil spring 60 is released and the slider member 71 performs the slide movement. The blade 30 then makes a cut in the skin due to the shaft portion 32 of the blade being movable along the guide groove 72m of the guide member 72, in conjunction with the slide movement of the slider member 71. The operation of the blade 30 is as described in detail above in "Operation Description of Blade According to First Embodiment" and "Operation Description of Blade According to Second Embodiment".

Due to the locking of the plunger 41 being released by the plunger lock piece 53 of the trigger member 50, the compression coil spring 60 is released and the plunger 41 moves inside the cylinder body 40a. That is, a change in volume is caused inside the cylinder body 40a and negative pressure is generated in the cylinder space SS and blade space BS. The operation of the negative pressure generation cylinder 40 is as described in detail above in "Operation Description of Negative Pressure Generation Cylinder".

As described in detail above in "Description of Trigger Member", the trigger member 50 preferably releases the locking of the blade 30 first and subsequently releases the locking of the negative pressure generation cylinder 40. Due to the trigger member 50 releasing the locking of the blade 30 and the locking of the negative pressure generation cylinder, it is possible to maintain the depth at which the blade 30 enters the skin at a fixed depth.

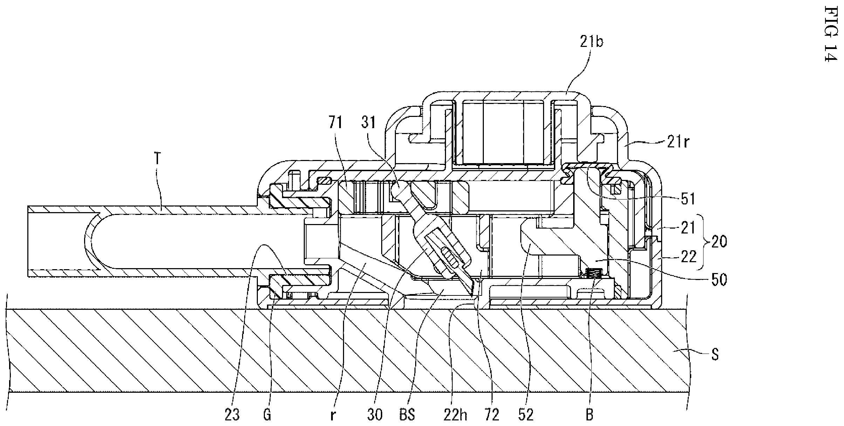

The collected blood travels along the flow path R in the housing 20 and is stored in the storage container T (see Figure 14). As a preferred aspect of the flow path R, the flow path R is preferably inclined with respect to the facing surface of the housing 20 that faces the skin S. More specifically, an end portion of the flow path R at the storage container T side is preferably inclined so as to move away from the facing surface. Due to the flow path R being inclined in this way, the collected blood can be appropriately guided to the storage container T.

The blood stored in the storage container T is preferably visible from the outside (Figure 15A). As an example, the storage container T is preferably made of a transparent material. According to such a configuration, it is possible to visually distinguish how much blood is stored in the storage container T.

After an adequate amount of blood is stored in the storage container T, the storage container T is removed from the blood collection device 1 (Figure 15B) and is transferred to an inspection body.

As described above, according to the blood collection device of the present disclosure, it is not necessary to individually prepare components for triggering the operation of the blade 30 and the plunger 41 since the trigger member 50 permits the blade 30 and the plunger 41 to be movable during blood collection by releasing the locking of both the plunger 41 and the blade 30. Therefore, it is possible to implement appropriate blood collection with a simple construction.

It should be noted that foregoing embodiments disclosed herein are by way of illustration in every respect and not to be taken by way of limitation. Therefore, the technical scope of the present disclosure is not construed only by the above-described embodiments, but defined based on the recitation of the claims and includes all modifications equivalent in meaning and scope to the claims. For example, the shape and/or the color scheme of the housing in the blood collection device or the blood collection and storage device may be changed depending on the application of the blood collection and/or per-customer basis. As an example, the housing may be changed to have a rectangular shape and/or a round shape depending on the application of the blood collection and/or per-customer basis, and/or the color scheme of the button of the housing may be changed depending on the application of the blood collection and/or per-customer basis.

Note that the blood collection device and the blood collection and storage device according to the present disclosure may include the following preferred aspects.

### First Aspect:

A blood collection device comprising a flow path guiding collected blood to a storage container comprising:
a housing;
a blade protruding from the housing and being movable to cut through a skin in one direction;
a negative pressure generation cylinder generating negative pressure in the housing due to a plunger being movable; and
a trigger member being accommodated in the housing and being actuated when blood collection starts, wherein
the trigger member is engaged with the plunger and the blade to restrict both of the plunger and the blade from being movable, and is disengaged with both of the plunger and the blade to permit the blade and the plunger to be movable.

### Second Aspect:

The blood collection device according to the above first aspect, wherein a compression coil spring being compressed in advance is used to make the plunger and/or the blade movable.

### Third Aspect:

The blood collection device according to the above second aspect, wherein the blade being movable is achieved by a movable body for allowing the blade to perform a shaft rotation through a biasing force generated from the compression coil spring.

### Fourth Aspect:

The blood collection device according to the above third aspect, wherein
the movable body comprises:
a slider member holding one end portion of the blade and being movable through the biasing force of the compression coil spring; and
a guide member with a guide groove accommodating a shaft portion for allowing the blade to perform the shaft rotation.

### Fifth Aspect:

The blood collection device according to the above fourth aspect, wherein the guide groove of the guide member is provided parallel to a direction of the housing toward the skin.

### Sixth Aspect:

The blood collection device according to the above fourth or fifth aspect, wherein the shaft portion is movable along the guide groove of the guide member.

### Seventh Aspect:

The blood collection device according to any one of the above fourth to sixth aspects, wherein
the slider member includes a diagonal groove, and
the one end portion of the blade is movable along the diagonal groove.

### Eighth Aspect:

The blood collection device according to any one of the above fourth to seventh aspects, wherein a direction of skin cutting by the blade is perpendicular to the direction of gravity when the blood collection device is adhering to the skin.

### Ninth Aspect:

The blood collection device according to the above fourth aspect, wherein on the slider member, the one end portion of the blade is movable in the same direction as the biasing direction of the compression coil spring.

### Tenth Aspect:

The blood collection device according to the above ninth aspect, wherein a direction of skin cutting by the blade is the direction of gravity when the blood collection device is adhering to the skin.

### Eleventh Aspect:

The blood collection device according to any one of the above second to tenth aspects, wherein a biasing direction of the compression coil spring used to make the plunger and/or the blade movable is perpendicular to the direction of the housing toward the skin.

### Twelfth Aspect:

The blood collection device according to any one of the above first to eleventh aspects, wherein an adhesive member is provided on a surface of the housing facing the skin.

### Thirteenth Aspect:

The blood collection device according to any one of the above first to twelfth aspects, wherein the trigger member disengages the plunger after disengages the blade.

### Fourteenth Aspect:

The blood collection device according to the above thirteenth aspect, wherein
the trigger member comprises:
a blade lock piece locking the movability of the blade; and
a plunger lock piece locking the movability of the plunger, and
a length of the blade lock piece is shorter than a length of the plunger lock piece.

### Fifteenth Aspect:

The blood collection device according to any one of the above first to fourteenth aspects, wherein negative pressure is generated by the negative pressure generation cylinder in a space in the housing accommodating the blade.

### Sixteenth Aspect:

The blood collection device according to any one of the above first to fifteenth aspects, wherein at least a circumference of the space accommodating the blade is hermetically sealed by an airtight retention member.

### Seventeenth Aspect:

The blood collection device according to any one of the above first to sixteenth aspects, wherein the negative pressure generation cylinder is disposed at both sides of the space accommodating the blade.

### Eighteenth Aspect:

The blood collection device according to any one of the above first to seventeenth aspects, wherein an attachment portion for attaching the storage container is provided at the direction of gravity side of the housing when the blood collection device is adhering to the skin.

### Nineteenth Aspect:

The blood collection device according to the above eighteenth aspect, wherein a flow path communicating with the attachment portion is inclined with respect to the surface of the housing facing the skin.

### Twelfth Aspect:

A blood collection and storage device comprising:
the blood collection device according to any one of the above first to nineteenth aspects; and
a storage container being positioned at the direction of gravity side when the blood collection device is adhering to the skin, and stores blood collected by the blood collection device.

### Industrial Applicability

The blood collection device and the blood collection and storage device according to the present disclosure are used for collecting blood from a subject or for storing the collected blood.

### Reference Signs List

- 1: blood collection device
- 20: housing
- 21: first body
- 21b: button
- 21r: ridge portion
- 22: second body
- 22a: adhesive member
- 22h: opening of second body
- 23: attachment portion (opening)
- 30: blade
- 31: end portion
- 32: shaft portion
- 40: negative pressure generation cylinder
- 40a: cylinder body
- 41: plunger
- 41a: disk portion
- 41b: shaft core
- 50: trigger member
- 51: abutment portion
- 52: blade lock piece
- 53: plunger lock piece
- 60: compression coil spring
- 70: movable body
- 71: slider member
- 71m: groove
- 71h: holding portion
- 72: guide member
- 72m: guide groove
- 80: adhesive member
- 90: airtight retention member
- B: spring
- G: gasket
- BS: blade space
- SS: cylinder space
- S: skin
- T: storage container
- R: flow path

## Claims

1. A blood collection device comprising a flow path guiding collected blood to a storage container comprising:
a housing;
a blade protruding from the housing and being movable to cut through a skin in one direction;
a negative pressure generation cylinder generating negative pressure in the housing due to a plunger being movable; and
a trigger member being accommodated in the housing and being actuated when blood collection starts, wherein
the trigger member is engaged with the plunger and the blade to restrict both of the plunger and the blade from being movable, and is disengaged with both of the plunger and the blade to permit the blade and the plunger to be movable.

2. The blood collection device according to claim 1, wherein a compression coil spring being compressed in advance is used to make the plunger and/or the blade movable.

3. The blood collection device according to claim 2, wherein the blade being movable is achieved by a movable body for allowing the blade to perform a shaft rotation through a biasing force generated from the compression coil spring.

4. The blood collection device according to claim 3, wherein
the movable body comprises:
a slider member holding one end portion of the blade and being movable through the biasing force of the compression coil spring; and
a guide member with a guide groove accommodating a shaft portion for allowing the blade to perform the shaft rotation.

5. The blood collection device according to claim 4, wherein the guide groove of the guide member is provided parallel to a direction of the housing toward the skin.

6. The blood collection device according to claim 4 or 5, wherein the shaft portion is movable along the guide groove of the guide member.

7. The blood collection device according to any one of claims 4 to 6, wherein
the slider member includes a diagonal groove, and
the one end portion of the blade is movable along the diagonal groove.

8. The blood collection device according to any one of claims 4 to 7, wherein a direction of skin cutting by the blade is perpendicular to the direction of gravity when the blood collection device is adhering to the skin.

9. The blood collection device according to claim 4, wherein on the slider member, the one end portion of the blade is movable in the same direction as the biasing direction of the compression coil spring.

10. The blood collection device according to claim 9, wherein a direction of skin cutting by the blade is the direction of gravity when the blood collection device is adhering to the skin.

11. The blood collection device according to any one of claims 2 to 10, wherein a biasing direction of the compression coil spring used to make the plunger and/or the blade movable is perpendicular to the direction of the housing toward the skin.

12. The blood collection device according to any one of claims 1 to 11, wherein an adhesive member is provided on a surface of the housing facing the skin.

13. The blood collection device according to any one of claims 1 to 12, wherein the trigger member disengages the plunger after disengages the blade.

14. The blood collection device according to claim 13, wherein
the trigger member comprises:
a blade lock piece locking the movability of the blade; and
a plunger lock piece locking the movability of the plunger, and
a length of the blade lock piece is shorter than a length of the plunger lock piece.

15. The blood collection device according to any one of claims 1 to 14, wherein negative pressure is generated by the negative pressure generation cylinder in a space in the housing accommodating the blade.

16. The blood collection device according to any one of claims 1 to 15, wherein at least a circumference of the space accommodating the blade is hermetically sealed by an airtight retention member.

17. The blood collection device according to any one of claims 1 to 16, wherein the negative pressure generation cylinder is disposed at both sides of the space accommodating the blade.

18. The blood collection device according to any one of claims 1 to 17, wherein an attachment portion for attaching the storage container is provided at the direction of gravity side of the housing when the blood collection device is adhering to the skin.

19. The blood collection device according to claim 18, wherein a flow path communicating with the attachment portion is inclined with respect to the surface of the housing facing the skin.

20. A blood collection and storage device comprising:
the blood collection device according to any one of claims 1 to 19; and
a storage container being positioned at the direction of gravity side when the blood collection device is adhering to the skin, and stores blood collected by the blood collection device.
